# EUROPEAN PATENT APPLICATION

(11) **EP 1 304 071 A2**
(43) Date of publication of application: **23.04.2003**
(21) Application number: 02257194.7
(22) Date of filing: 17.10.2002
(51) Int. Cl.: A61B 5/00

(54) **Remote monitoring system and method**

(30) Priority: 17.10.2001 GB 0124995
(71) Applicant: Medical Tracking Ltd., East Sussex BN8 5AT (GB)
(72) Inventor: Abbott, Michael John, Barcombe, East Sussex NB85AT (GB)
(74) Representative: Howe, Steven

(57) **Abstract**

System and method for remote monitoring of a medical parameter of a patient (12). The medical parameter is measured, using a measuring means (21). The measured parameter is compared with a range of values for the parameter. A signal indicative of the measured value of the parameter is transmitted to a base station (13) after a first time period, or in response to a request. If the parameter is determined to be outside a range of acceptable values, the signal is transmitted to the base station at a second predetermined time period, a second predetermined time period being less than the first predetermined time period.

## Description

The present invention relates to a system for the remote monitoring of one or more medical parameters of a patient, and to a method for such remote monitoring.

It is usual for a patient to remain in hospital after treatment so that the patient can be kept under observation in case the condition of the patient deteriorates. In this case, monitoring equipment may be connected to the patient to monitor the patient's vital signs, such as blood pressure, pulse rate, respiration and glucose level. An indication of the measured parameters may be provided to medical personnel so the patient may be kept under observation. In some cases, when the measured parameters fall outside a predetermined range, a warning may be provided to the medical personnel caring for the patient, for example in the form of an audible alarm.

For some patients with a long term illness or condition, it may not be desirable for the patient to remain in hospital. The patient may instead prefer to be at home. In this case, it is known for the monitoring equipment measuring the vital signs of the patient to be connected, for example using a modem and telephone line, to send the measured parameters to the hospital so that the patient may be monitored remotely. This may be possible where it is desired to monitor the condition of the patient over a longer period, rather than trying to monitor sudden changes in the condition of the patient. In these circumstances, it is necessary for the patient to remain connected to the monitoring apparatus which in turn is connected to the telephone line to communicate the measured information to the hospital.

Many systems are known by which information from a remote station is communicated to a base station via a satellite. Examples of such systems include those for monitoring the location of a unit, for example of a vehicle such as a car or a boat, or a package. This allows the location of a stolen vehicle or a lost package to be identified. Such systems usually require that a communications system is provided on the unit to be monitored, and this is activated when the vehicle is reported as being stolen or when the package is to be located, and in this case the system transmits a signal indicative of its location. Such systems are therefore expensive, requiring a Global Positioning System (GPS) unit for determining the position of the unit, and tend to be large.

According to a first aspect of the present invention, a system for remotely monitoring a medical parameter of a patient comprises:
a measuring means for measuring a medical parameter of a patient;
a comparator for comparing the measured parameter with a range of values for the parameter;
a transmitter for transmitting a signal indicative of the measured value of the parameter to a base station, wherein the signal indicative of the measured value of the parameter is transmitted to the base station after a first time period or in response to a request, and wherein, if it is determined that the measured parameter is outside the range of values for the parameter, the signal indicative of the measured value of the parameter is transmitted to the base station after a second predetermined time period, the second time period being less than the first time period or before the request for information.

With the system of the present invention, the measuring means determines the desired medical parameter for the patient, for example the blood pressure or the pulse rate of the patient. The measured parameter is then compared with a range of acceptable values for the parameter. It is hoped that in most cases, the measured value will fall within a range of acceptable values for the parameter. In this case, a signal indicative of the measured value is transmitted to the base station, which may be at a hospital or other place where medical personnel can monitor the measured values, after a first time period. This first time period is typically a long time period, for example about twelve hours. However, when the measured parameter falls outside the range of acceptable values, there are more frequent transmissions of the measured parameter, for example once every minute, or once each time a measurement of the parameter is made. In this way, when the measured parameter is considered to be acceptable, there are occasional transmissions to the base station. This is sufficient since the measured values are in the range considered acceptable, and therefore no action will generally need to be taken, and so the actual values are not generally of great importance. However, when the measured value is outside the range of acceptable values, and therefore it is more important for these to be considered by the medical personnel looking after the patient, the measured values are transmitted to the base station more frequently. Therefore, an up-to-date measurement is made available to the medical personnel so they are in a better position to determine what, if any, action needs to be taken. By reducing the frequency of transmissions when the measured values are within an acceptable range, the amount of information that needs to be transmitted to the base station is reduced. This is of advantage as it decreases the power requirement for the device monitoring the parameter of the patient, and as it decreases the amount of information that is transmitted to the base station, and therefore allows a greater number of remote monitoring devices to use the same communication channel to the base station than would be the case if more frequent transmissions were made.

In a preferred example, the signal indicative of the measured parameter is only transmitted at the second time interval if there are a number of measurements falling outside the range of acceptable values.

When it is determined that the measured parameter falls outside the range of acceptable parameters, the measured values may continue to be transmitted at the second rate either for a predetermined number of transmissions, or for a predetermined time period, or until the measurements again fall within the acceptable range.

The communication between the remote station monitoring the parameter of the patient and the base station is preferably made over a wireless channel, such that the patient can move freely without being tied to a fixed communication channel. In particular, the channel may be a radio channel, a cell-phone based channel, and most preferably a satellite link.

It is possible that the measuring means measures more than one medical parameter of the patient. For example, both the pulse rate and the blood pressure of the patient may be monitored. When a transmission is made to the base station, it is preferred that all the measured values for all of the measured parameters are transmitted. In this case, each of the measured parameters may be compared with a range of acceptable parameters.

When a transmission is made, the signal may be indicative of the last measurement made for a parameter, the minimum value measured since the last transmission, the maximum value measured since the last transmission, and/or the average value measured since the last transmission. This information may be useful in assessing the condition of the patient.

Preferably, the transmission includes an identification of the patient to which the transmission applies. This is especially important when there are a number of patients monitored by the same base station. The transmission may also include an indication of the location of the patient, for example using a GPS system. In this way, in the event that the patient needs to be located, for example to treat the patient in view of the medical parameters measured, the patient may easily and accurately be located so that medical personnel to treat the patient may be sent to the patient quickly.

The ranges of acceptable values for the or each of the measurements may be varied. For example, depending on the medical condition of the patient, the range of acceptable values may be determined and the comparator set accordingly. In this case, the device may include a microprocessor in which the range of acceptable values may be programmed before the patient is released. Alternatively or additionally, the range of acceptable values may be varied remotely during use. This is preferably achieved by a signal transmitted from the base station to the remote monitoring device, and for this reason it is preferred that the communication channel between the remote monitoring device and the base station is a two-way communication channel. In this case, the timing of the transmissions may be controlled and varied remotely. For example, a signal may be sent from the base station to request a transmission of the measured values from the remote monitoring station. Further, the base station may change the periods when the transmissions are made from the remote monitoring station. In this case, the period may be increased over time whilst the measured values remain within the range of acceptable values.

When the measured value of the medical parameter falls outside the range of acceptable values, it is preferred that an alarm is sounded at the base station and/or at the measuring unit. In this way, if an unexpected or unacceptable parameter is determined, a warning is given so that appropriate action may be taken.

The measurements transmitted to the base station may be stored for subsequent analysis. In this case, the results may be accessible by the patient, for example over the Internet or by other means of communication. Alternatively or additionally, the measurements may be stored in the measuring device, and these results may be accessed from the measuring device, for example by downloading these to a computer or other equipment, or by the provision of a display on the measuring device that can display the measured values.

According to a second aspect of the present invention, a method for remotely monitoring a medical parameter of a patient, comprises the steps of:
measuring a medical parameter of a patient;
comparing the measured parameter with a range of values for the parameter;
transmitting a signal indicative of the measured value of the parameter to a base station, wherein the signal indicative of the measured value of the parameter is transmitted to the base station after a first time period or in response to a request, and wherein, if it is determined that the measured parameter is outside the range of values for the parameter, the signal indicative of the measured value of the parameter is transmitted to the base station after a second predetermined time period, the second time period being less than the first time period or before the request for information.

Further preferred features of the method of this aspect of the present invention correspond to the preferred features of the system as described above.

The present invention will now be described, by way of example, with respect to the accompanying drawings, in which:
Figure 1 shows the overall system for remote monitoring of a patient; and,
Figure 2 shows a schematic drawing of the remote monitoring unit.

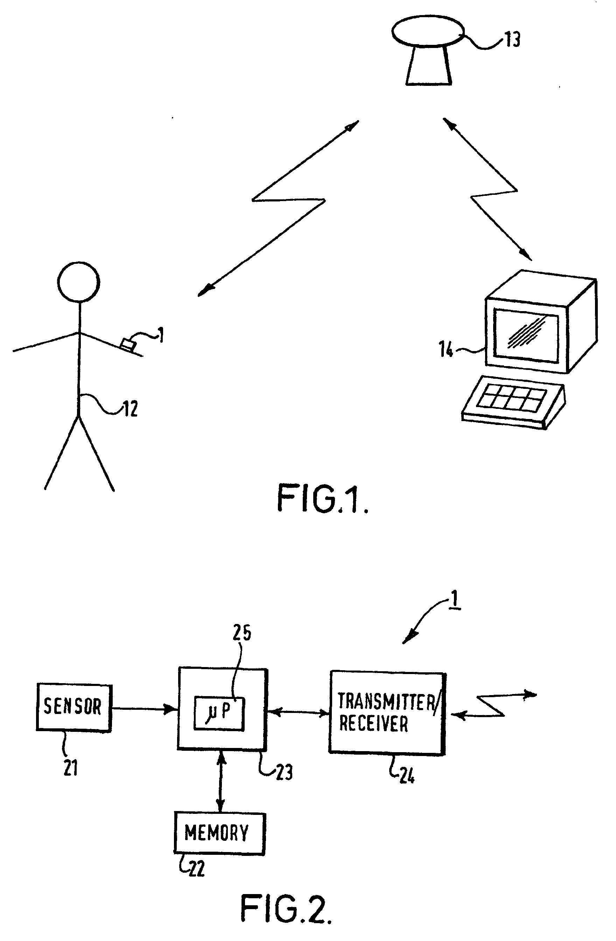

As shown in Figure 1, a patient 12 whose medical condition is to be monitored wears a remote measuring device 1. The measuring device 1 transmits a signal indicative of the measured parameter of the patient, via a satellite 13 to a base station 14.

The monitoring device 1 is shown schematically in Figure 2, and includes a sensor or measuring device 21, a microprocessor 23 with associated memory 22, and a transmitter/receiver 24. The measuring device 21 may include any form of device capable of measuring a desired medical parameter of the patient 12, for example to measure the blood pressure and/or the pulse rate of the patient 12, and producing an output signal indicative of the measured value. Various suitable devices are known, for example the (HD-505) available from Samsung that measures both the blood pressure and the pulse rate of the patient, or the (DREAM BEAM ANALYSER) available from Futrex that measures the blood glucose level of the patient non-invasively. Such known devices are able to output signals indicative of the measured results to an interface, for example using an RS232 output port. The measuring device is preferably small and lightweight, allowing this to be worn continually by the patient, for example on the patient's wrist or around the patient's waist. The measuring device 12 is battery powered, such that the device does not need to be permanently connected to a mains source of power. The battery may be rechargeable to extend the life of the device, or the battery may be replaceable.

In a preferred example of the present invention, the measured parameters are transmitted to the base station 14 via a satellite. However, the signals may be transmitted to the base station 14 over other communications channels, for example through a cell phone or radio network. One possible satellite link is the Inmarsat D+, although other satellites such as Globalstar or ICO may equally be used. These systems do not give global positioning information, however systems giving this facility may be used if this feature is required. A suitable transmitter/receiver 24 is provided to transmit data to the base station 14 via the satellite 13, and to receive information from the base station 14 via the satellite 13. Where the satellite 13 is the Inmarsat D+, suitable transceivers include those available from JRC (JRC-1970) and from Skywave (DMR-200). The JRC Inmarsat D+ communicator device has an RS232 hardware interface permitting full duplex communication at a rate of 1200 bits per second.

As shown in Figure 2, the outputs from the measuring device 21 indicative of the measured medical parameter are input to an interface 23, including a microprocessor 25. A memory 22 is coupled to the microprocessor 22, and the memory stores data for the control of the interface 23. The memory 22 includes data representative of the timing for transmissions of signals to the base station, and the ranges of acceptable values for the parameters measured.

In one example, the measuring device 21 periodically determines the pulse rate of the patient, for example once every three minutes or once a minute. This information is transmitted to the interface 23. The microprocessor 25 compares the measured value of the pulse rate with an upper and lower acceptable limit stored in the memory 22. In the case that the measured pulse rate falls between the upper and lower limits, the actual measured value is stored in memory, and the process is repeated for subsequent measurements. In this case, the interface 23 will cause the communicator 24 to transmit information to the base station 14 after a predetermined period, for example after 12 hours. The transmission may transmit any desired information, but the transmission will typically include an identification of the patient, the maximum and the minimum values of the measurements since the last transmission and the average value of the measured parameter since the last transmission. The information may also include other statistical information determined by the microprocessor based on the measurements, and may include timing information indicating the time of the transmission, the time of the minimum and maximum measurements and other relevant information.

In the event that the measured value for the medical parameter falls outside the predetermined range of acceptable values, it is determined that the information is important and therefore needs to be brought to the attention of the medical personnel treating or looking after the patient. Therefore, rather than waiting for the next scheduled transmission, an additional transmission is made. In this case, it is possible that the measurement outside the range of acceptable values is a single rogue measurement that is not repeated. Accordingly, in one example, the additional transmission will only be made if there are a number of successive measurements outside the range of acceptable values. In other cases, it may be considered that an additional transmission is require when ever any measurements are made falling outside the range of acceptable values. Whilst a single additional transmission may be made, it is preferred that a number of transmissions are made at an increased rate, thereby transmitting a number of subsequent measurements. This allows the medical personnel treating the patient to determine whether the abnormal or unexpected result was an isolated result, whether this abnormal condition continues, and whether the abnormal conditions rectifies itself. The more frequent transmissions may continue for a predetermined time period or a predetermined number of transmissions, or until the measurements fall within the range of acceptable values.

The rate at which more frequent transmissions are made normally correspond to the rate at which the measurements are taken, for example once every minute. However, a different rate may be set. Further, the rate of the increased frequency of transmissions may decrease as the measured value returns to be within the predetermined range.

The rate of transmissions following the measurement of a parameter outside the range of acceptable values, and the range of acceptable values may be varied during the operation of the monitoring means. The variation may be made by programming of the microprocessor, in which case the microprocessor gives the measuring device a degree of intelligence. Alternatively or additionally, the variation may be controlled in response to a signal received from the base station. In this case, the base station may include a degree of intelligence so this can determine the need to change the control parameters. However, it is usual that an operator, for example trained medical personnel treating the patient instruct the variation. For example, when the patient is first discharged, the medical personnel may set a short normal period for transmissions from the measuring device, and may set a large range of acceptable values. As the patient's condition improves, and less monitoring is required, the period for normal transmissions may be increased, so that transmissions are made less often. At the same time, the range of acceptable values may be decreased as it is expected that the patient's medical condition will become more regular and fall within a smaller range of values. Any changes in the operation of the measuring device are transmitted from the base station 14 via the satellite 13 to the remote monitoring device 1. To avoid unauthorised changes being made, the transmissions instructing the changes may be password controlled, such that the transmission will only be made if the correct password is entered. The current setting of the remote monitoring unit 1 may be transmitted to the base station 14 on request to ensure that the setting is correct.

The base station will typically be accessible to the trained medical personnel caring for the patient. In one example, the base station can be provided at a hospital where the same team of medical personnel is able to monitor the parameters of a number of their patients. However, to increase the applicability of the system, it is preferred that all measurements from a number of patients are received by a general base station, and this transmits the measurements for individual patients to the medical personnel caring for these patients. For example, the system may include a single base station that receives the transmissions from many measuring devices via a satellite. Then, details of the measurements for individual patients are sent to computers at the hospitals or other locations where the medical personnel caring for each patient are located. This communication from the base station to a local station may be made by any suitable means, which may include transmissions over the Internet. In this way, medical personnel may, at any time, log-on and receive the results of the measurements for individual patients of interest. The information may be transmitted by e-mail to any desired address. In any case, real time monitoring of the patient's condition will be available.

## Claims

1. A system for remotely monitoring a medical parameter of a patient comprising:
a measuring means for measuring a medical parameter of a patient;
a comparator for comparing the measured parameter with a range of values for the parameter;
a transmitter for transmitting a signal indicative of the measured value of the parameter to a base station, wherein the signal indicative of the measured value of the parameter is transmitted to the base station after a first time period or in response to a request, and wherein, if it is determined that the measured parameter is outside the range of values for the parameter, the signal indicative of the measured value of the parameter is transmitted to the base station after a second predetermined time period, the second time period being less than the first time period or before the request for information.

2. A system according to Claim 1, in which the first time period is about twelve hours.

3. A system according to Claim 1 or Claim 2, in which the second time period corresponds to the time for measuring the medical parameter.

4. A system according to any one of the preceding claims, in which the second time period is about one minute.

5. A system according to any one of the preceding claims, in which the signal indicative of the measured parameter is only transmitted at the second time interval if there are a number of measurements falling outside the range of acceptable values.

6. A system according to any one of the preceding claims, in which when it is determined that the measured parameter falls outside the range of acceptable parameters, the measured values may continue to be transmitted at the second rate either for a predetermined number of transmissions, or for a predetermined time period, or until the measurements again fall within the acceptable range.

7. A system according to any one of the preceding claims, in which the communication between the remote station monitoring the parameter of the patient and the base station is made over a wireless channel.

8. A system according to Claim 7, in which the wireless channel includes a satellite.

9. A system according to any one of the preceding claims, in which more than one medical parameter of the patient.

10. A system according to any one of the preceding claims, in which the signal transmitted is indicative of the last measurement made for a parameter, the minimum value measured since the last transmission, the maximum value measured since the last transmission, and/or the average value measured since the last transmission.

11. A system according to any one of the preceding claims, in which the transmission includes an identification of the patient to which the transmission applies.

12. A system according to any one of the preceding claims, in which the range of acceptable values for the or each of the measurements may be varied.

13. A system according to claim 12, in which the device includes a microprocessor in which the range of acceptable values may be programmed.

14. A system according to Claim 12 or 13, in which the range of acceptable values is varied remotely.

15. A system according to Claim 14, in which the range of acceptable parameters is varied remotely by a signal transmitted from the base station to the remote monitoring device.

16. A system according to any one of the preceding claims, in which a signal is sent from the base station to request a transmission of the measured values from the remote monitoring station.

17. A system according to any one of the preceding claims, in which the periods when the transmissions are made from the remote monitoring station can be controlled.

18. A method of remotely monitoring a medical parameter of a patient, the method comprising the steps of:
measuring a medical parameter of a patient;
comparing the measured parameter with a range of values for the parameter;
transmitting a signal indicative of the measured value of the parameter to a base station, wherein the signal indicative of the measured value of the parameter is transmitted to the base station after a first time period or in response to a request, and wherein, if it is determined that the measured parameter is outside the range of values for the parameter, the signal indicative of the measured value of the parameter is transmitted to the base station after a second predetermined time period, the second time period being less than the first time period or before the request for information.
